# EUROPEAN PATENT SPECIFICATION

(11) **EP 1 777 309 B1**
(45) Date of publication and mention of the grant of the patent: **06.10.2010**
(21) Application number: 05770571.7
(22) Date of filing: 10.08.2005
(51) Int. Cl.: C22C 9/00, C22C 9/04, C22C 18/00, C22C 18/02, C22C 1/02, B22D 1/00, B22D 21/00, B22D 27/20, C22C 1/03, C22C 30/02, C22C 30/06

(54) **USE OF MASTER ALLOY FOR MODIFYING COPPER ALLOY AND CASTING METHOD USING THE SAME**
VERWENDUNG VON VORLEGIERUNG ZUR MODIFIZIERUNG VON KUPFERLEGIERUNG UND GIESSVERFAHREN DAMIT
UTILISATION D'ALLIAGE MÈRE POUR MODIFICATION D'ALLIAGE DE CUIVRE ET PROCÉDÉ DE MOULAGE UTILISANT CET ALLIAGE

(30) Priority: 10.08.2004 JP 2004233952
(43) Date of publication of application: 25.04.2007
(73) Proprietor: Mitsubishi Shindoh Co., Ltd., Kita-shinagawa Shinagawa-ku Tokyo (JP)
(72) Inventor: OISHI, Keiichiro, Yao-shi, Osaka 581-0032 (JP)
(74) Representative: HOFFMANN EITLE
(86) International application number: PCT/JP2005/014678
(87) International publication number: WO 2006/016614

(56) References cited:
- JP-A- 4 099 837
- JP-A- 4 099 837
- JP-A- 52 134 811
- JP-A- 56 090 944
- JP-A- 56 090 944

## Description

### Technical Field

The present invention relates to the use of a master alloy for casting a modified copper alloy having refined grains, which is used in a casting method such as continuous casting, semi-solid metal casting, sand casting, permanent mold casting, low pressure die casting, die casting, lost wax casting, up casting, squeeze, centrifugal casting or the like, and also relates to a method of casing a modified copper alloy using the same.

### Background Art

Since the grain refinement of a copper alloy is very effective in improving 0.2% proof strength (a strength when permanent distortion reaches 0.2%, hereinafter referred to as simply 'proof strength') or the like, it is strongly desirable to refine grains of a copper alloy. For example, the proof strength is proportional to one over the square root of the grain size D (D^{-1/2}) according to the Hall-Petch theory (see E. O. Hall, Proc. Phys. Soc. London. 64 (1951) 747. and N. J. Petch, J. Iron Steel Inst. 174 (1953) 25.).

Basically, the grains of a copper alloy have been refined as follows:
(A) the grains are refined during the melt-solidification of the copper alloy, or
(B) the grains are refined by processing or heating the copper alloy (ingot such as slurry or the like; casting
including die casting or the like; and hot forged parts or the like), in which stacking energy such as distortion energy or the like acts as a driving force.
As methods of refining the grains like (A) in the prior art, (a) to (d) have been proposed.
(a) Crystallized substances or the like are made to act as crystal nuclei by adding grain refining elements such as Ti, Zr or the like (Introduction of effective heterogeneous nuclei) (for example, see Patent document 1).
(b) Homogeneous nuclei are generated by pouring a molten alloy within an extremely narrow temperature range and thus subjecting the molten alloy to super-cooling.
(c) Facilitating the generation of crystal nuclei or cutting the arms of grown dendrites (tree-like crystal) by using an electromagnetic induction agitator or steering (a device for stirring the molten alloy); usually combined with method (b).
(d) Rapid solidification technique by die casting or the like or solidifying a casting locally and rapidly by using a chilling block.
In the above methods, the molten alloy is solidified before the dendrites are grown, whereby the grains are refined.

In addition, as the methods of refining the grains after casting like (B) in the prior art,
(e) Part of distortion energy provided by adequate processes (rolling, drawing, forging or the like) on a melt-solidified alloy material such as ingot or the like is accumulated in a metal, and the energy accumulation brings the increase in re-crystallization nuclei, whereby the grains are refined by using the energy as a driving force (see Patent document 2).
(f) Melt-solidified alloy material such as ingot or the like is provided with proper distortion energy and then heated, whereby the accumulated energy released by the heating leads to re-crystallization.

Patent document 1: JP-A-2004-100041
Patent document 2: JP-A-2002-356728

However, in the method (a), a large amount of the grain refining elements should be used, and the large amount of the grain refining elements can have an adverse influence on the inherent features of a copper alloy. That is, even though the components of the copper alloy are selected and determined to make the copper alloy have the features suitable for the usage or the like, when the grains of the copper alloy composed of the above-mentioned components (hereinafter referred to as 'copper alloy to be modified') are refined by method (a) in order to produce a grain-refined copper alloy (referred to as 'modified copper alloy'), the adverse influence of the large amount of the grain refining elements on the inherent features of the copper alloy to be modified is bigger than the feature-improving effect or feature-enhancing effect obtained by the grain refinement of the copper alloy to be modified, whereby the features of the modified copper alloy cannot be improved or enhanced as a whole.

In addition, since both methods (b) and (c) take a large space or long time, the methods are not suitable for a small and complex-shaped casting as well as a large-scaled and great quantity of ingots, which are formed in a predetermined shape by a continuous operation. Furthermore, the grains cannot be refined by the methods as effectively as the above problems can be ignored, whereby the methods have few industrial merits. Still furthermore, a method of (d) has the following problem: that is, the rapid solidification technique such as die casting or the like can be applied to a limited range of solidified shapes or producing procedures, and the rapid solidification technique using a chilling block solidifies a casting locally, whereby the technique can be installed to limited places and refine the grains to a low degree.

Still furthermore, in methods (e) and (f), which are basically different from methods (a) to (d), in which the grains are refined during the melt-solidification, the grains are refined by providing energy to an alloy after the melt-solidification, and a machine for providing energy (for example, rolling machine, drawing machine or forging machine) is required, whereby energy or initial and running cost for the grain refinement would rise significantly.
JP 56 090944 A discloses alloys for a wire cut electrospark machining electrode which contain 11 to 40% Zn and 0.1 to 3% Zr with the balance being Cu.

It is an advantage of an aspect of the invention to provide a method of casting a modified copper alloy capable of refining grains during the melt-solidification of the copper alloy without the problems in the related art being induced.

### Disclosure of the Invention

Grains can be refined during the melt-solidification of an alloy when primary crystals are generated from a molten liquid much faster than the growth of dendrite crystals.
After passionate investigations, the present inventors found out that when an extremely small amount of Zr is added to a copper alloy in the presence of P and the ratio of P/Zr is in a proper range, the generation of alpha phases, primary crystals, is facilitated considerably, whereby the grains are refined remarkably during the melt-solidification. In addition, it is found that when peritectic or eutectic reaction occurs during solidification and beta phases are crystallized around the primary alpha phases, the grains are further refined. Furthermore, it is also found that when the beta phases are transformed into kappa, gamma, delta, and mu-phases in the alpha phase matrix by the reaction in solid phases, the grains are much further refined.

However, Zr is an active and high melting point metal, whereby it is difficult to control the amount of Zr in a narrow range. In addition, even when the predetermined amount of Zr is added to a copper alloy, if Zr in the copper alloy is oxidized or sulphurized, such oxidized or sulfurized Zr cannot make any contribution to the grain refinement. On the other hand, when a large amount of Zr is added, the effect of Zr to refine the grains is saturated, and further, Zr does not contribute to the grain refinement, whereby the grain size increases, and the features such as electric thermal conductivities or the like deteriorate. Furthermore, copper alloy members containing a large amount of Zr can generate a great amount of oxide and sulfide according to the melting atmosphere and the type or state of raw materials when they are re-melted for recycling (various manufacturing processes (material - commercialization), disposed products or the like), whereby high quality castings cannot be made.

Still furthermore, even though the amount of Zr can be controlled in an extremely narrow range with relative easiness if non-contaminated materials are melted in a melting furnace having special equipment or the like that makes non-oxidation atmosphere and vacuum, such special equipment is expensive, and a great amount of energy and time must be consumed in order to use the equipment. Still furthermore, it is also found that the way of adding Zr needs to be studied in order for a base material to contain the minimum necessary amount of Zr, which can refine the grains even when the alloy is cast at an end user level.

After passionate investigations, the inventors found out a casting method, through which Zr can remain in a molten liquid without being oxidized nor sulfurized, even when the furnace having a special equipment or the like is not used. That is, the inventors found out that Zr needs to be added in the form of Cu - Zn - Zr or Cu - Zn - P - Zr master alloy so that it can remain in the molten alloy without being oxidized nor sulphurized in order to refine the grains, while, in general, Zr is added to a molten copper alloy in the form of Cu - Zr.

The invention provides the use of a master alloy for casting a modified copper alloy, wherein the master alloy is a Cu-Zn-Zr alloy comprising Cu: 40 to 80%, Zr: 0.5 to 35% and optionally at least one element selected from the group consisting of Mg: 0.01 to 1%, Al: 0.01 to 5%, Sn: 0.1 to 5%, B: 0.01 to 0.5%, Mn: 0.01 to 5% and Si: 0.01 to 1%, and the balance of Zn, wherein the amount of Zn is larger than the amount of Zr, or a Cu-Zn-Zr-P alloy comprising Cu: 40 to 80%, Zr: 0.5 to 35%, P: 0.01 to 3% and optionally at least one element selected from the group consisting of Mg: 0.01 to 1%, Al: 0.01 to 5%, Sn: 0.1 to 5%, B: 0.01 to 0.5%, Mn: 0.01 to 5% and Si: 0.01 to 1%, and the balance of Zn, wherein the amount of Zn is larger than the amount of Zr.
Particularly, it is more preferable that Cu occupy 50 to 65 wt.% and Zr occupy 1 to 10 wt.% in the master alloys since the master alloys have a low-melting point and can be rapidly melted into a molten alloy. (In the present specification, it should be understood that "%" means "wt.%".) When a master alloy of the invention is used, metal Zr as well as P can exist in the molten alloy during solidification without influence of oxidation or sulfurization even when a small amount of Zr is added. Accordingly, primary alpha phases are crystallized and grains are easily refined.

According to the invention, when manufacturing a copper alloy by casting molten copper alloy containing Zr and P, it is possible to cast a modified copper alloy by adding at least Zr in the form of Cu - Zn - Zr or Cu - Zn - Zr - P master alloy. In the above-mentioned casting methods according to the invention, a concentration of metal Zr in the molten copper alloy, which is required for grain refinement, is easily controlled in the range of 5 ppm or more and preferably 20 to 500 ppm. It is, therefore, possible to efficiently crystallize primary alpha phases and to efficiently refine grains.

### Brief Description of the Drawings

Fig. 1 is a view showing a macro-structure of 76Cu - 3Si - 21Zn casting, which is cast by using the master alloy of Sample No. 1 (62Cu - 3Zr - 35Zn) in Table 1, observed by a magnifying glass of 7.5 times magnification;
Fig. 2 is a view showing the micro-structure of 76Cu - 3Si - 21Zn casting, which is cast by using the master alloy of Sample No. 1 in Table 1, observed by a metallurgical microscope;
Fig. 3 is a view showing the macro-structure of 76Cu - 3Si - 21Zn casting, which is cast by using the master alloy of Sample No. 13 (50Cu - 50Zr) in Table 1, observed by the magnifying glass of 7.5 times magnification; and
Fig. 4 is a view showing the micro-structure of 76Cu - 3Si - 21Zn casting, which is cast by using the master alloy of Sample No. 13 in Table 1, observed by the metallurgical microscope.

### Best Mode for Carrying Out the Invention

The invention provides the use of a master alloy for casting a modified copper alloy, wherein the master alloy is a Cu-Zn-Zr alloy comprising
Cu: 40 to 80%, Zr: 0.5 to 35% and optionally at least one element selected from the group consisting of Mg: 0.01 to 1%, Al: 0.01 to 5%, Sn: 0.1 to 5%, B: 0.01 to 0.5%, Mn: 0.01 to 5% and Si: 0.01 to 1%, and the balance of Zn, wherein the amount of Zn is larger than the amount of Zr,
or a Cu-Zn-Zr-P alloy comprising
Cu: 40 to 80%, Zr: 0.5 to 35%, P: 0.01 to 3% and optionally at least one element selected from the group consisting of Mg: 0.01 to 1%, Al: 0.01 to 5%, Sn: 0.1 to 5%, B: 0.01 to 0.5%, Mn: 0.01 to 5% and Si: 0.01 to 1%, and the balance of Zn, wherein the amount of Zn is larger than the amount of Zr.
Hereinafter, the reason why each component should be in a limited range will be described.

Cu: Since the invention relates to the use of a copper alloy master alloy, Cu is a main element. However, the melting point does not decrease as much as desired when Zr is added to pure Cu (melting point: 1083°C) (therefore, it will take a long time to melt the master alloy: consequently, the effective amount of Zr decreases, and the formation of zirconium oxide is facilitated in the alloy, whereby Zr becomes totally ineffective). In addition, copper alone cannot prevent the loss of Zr during the melting of the master alloy and the formation of zirconium oxide in the alloy, therefore, another additives are required to prevent the oxidation loss and sulphurization loss of Zr and the formation of zirconium oxide in the alloy. Furthermore, even when the other alloy element (Zn) is added, if Cu occupies more than 80%, the above three problems (melting point, the loss of Zr, the absence of effective Zr) cannot be solved satisfactorily. However, when no Zn is contained in the alloy to be modified, a master alloy used for such alloy must consist of majority of Cu and minority of Zn, otherwise, the amount of Zn in the modified alloy may exceed the limit allowed for impurities.

Meanwhile, the reason why the minimum amount of Cu is defined as 40% is that, when the amount of Cu does not reach 40%, the melting point (liquidus line temperature) seldom decreases and, conversely, high melting point zirconium oxide is generated. In addition, when the amount of Cu decreases, that is, the amount of the remainder Zn becomes excessive, too much Zn is evaporated and thus the melting temperature does not decrease during the manufacture of a master alloy, whereby it is difficult to manufacture the master alloy.

Zr: Zr is an important element for grain refinement during the solidification. The melting point (liquidus line temperature) of a metal decreases when the metal is alloyed. That is, the melting point of Zr is 1850°C, and the melting point of Cu - Zr intermediate alloy is in the range of 1000 to 1120°C. However, an average copper alloy has the liquidus line temperature in the range of 870 to 1050°C, the melting temperature in the range of 950 to 1200°C, and the pouring temperature in the range of 890 to 1150°C. The melting point of a master alloy needs to be equal to or lower than the liquidus line temperature of such copper alloy. In addition, the amount of Zr begins to decrease as soon as Zr is melted in the oxidation atmosphere. Therefore, if the melting takes a long time, the amount of Zr cannot reach the predetermined amount. Therefore, it is preferable that the melting point be as low as possible.

The minimum amount of Zr is defined as 0.5%, which is 100 times of the required amount 0.005%, in consideration of economic burden, time and effort for charging Zr into a molten alloy or the like. Even though it is preferable that the maximum amount of Zr be as high as possible, the melting point of the alloy does not decrease. The maximum amount of Zr is, therefore, defined as 35% in order to make the melting point equal to or lower than the liquidus line temperature. The amount of Zr is preferably in the range of 1 to 20%, more preferably in the range of 1 to 10%, and most preferably in the range of 2 to 6%.

Zn: With the addition of Zn, a low melting point Zr - Zn - Cu intermetallic compound can be formed, whereby the melting point of such intermetallic compound is lowered than that of the matrix. Next, a molten alloy contains oxygen, and some oxygen forms zinc oxide before the oxygen forms zirconium oxide, whereby the amount of oxygen in the molten alloy decreases so as to prevent Zr from being oxidized and, consequently, to prevent the loss of Zr amount. Therefore, the amount of Zn is larger than that of Zr, depending also on the concentration of oxygen in the molten alloy. It is preferable that the amount of Zn be twice or more than that of Zr, and most preferable that the amount of Zn be three times or more than that of Zr. However, it is required to adjust the amount of Zr and Zn properly on the basis of the amount of Zn that can be contained as an impurity in a copper alloy to be modified, such as Cu-Sn, which does not contain Zn as a necessary element. Therefore, it is most preferable that the master alloy contain 50 to 65% of Cu, 1 to 10% (2 to 6%) of Zr, and the remainder Zn. In this case, the melting point reaches the lowest, and, since more amount of Zn is contained than that of Zr in the alloy, more amount of Zn is melted than that of Zr when Zr in the master alloy is melted, whereby the oxidation loss of Zr and the formation of zirconium oxide can be prevented.

P: P is an essential element. P can be added in a form of Cu - P alloy as well as in a form of Cu - Zr - P - Zn master alloy. The amount of P should be in the range of 7 to 20%, preferably 10 to 15% in the case of Cu - P alloy and 0.01 to 3% in the case of Cu - Zr - P - Zn master alloy. However, the amount of P should be adjusted to meet the following ratio of P/Zr at the melt-solidification. Meanwhile, it is preferable that the Cu-Zr-P-Zn master alloy contain, particularly, 50 to 65% of Cu and 1 to 10% of Zr, since the melting point decreases, and the master alloy can be melted into molten alloy quickly.

It is preferable that the master alloys according to the invention further contain at least one element selected from the group consisting of Mg: 0.01 to 1%, Al: 0.01 to 5%, Sn: 0.1 to 5%, B: 0.01 to 0.5%, Mn: 0.01 to 5%, and Si: 0.01 to 1%.

These elements further lower the melting point of the Zr intermetallic compound and the melting point of Cu - Zn matrix. In addition, the elements prevent the oxidation sulphurization loss of Zr. Mg, Mn and Al prevent the sulphurization loss. The reason why the elements should be in the limited range is as follows: the minimum amount is the necessary amount to prevent the oxidation loss of Zr, and conversely, over the maximum amount raises the melting point, whereby no better effect can be found even when the element is added more than necessary.

Meanwhile, if 0.005 mass% or more of Mg is contained in a molten alloy before charging Zr, the component S in the molten alloy is removed or fixed in the form of MgS. However, if the excessive amount of Mg is added to a molten alloy, Mg is oxidized like Zr, whereby casting defects such as oxide inclusion or the like happen. Mn also removes the component S, even though not as much as Mg. Sn refines the grains remarkably in the presence of Zr and P, even though Sn alone can refine the grains to a small degree. Sn improves mechanical properties (strength or the like), corrosion resistance, and wear resistance, and works to cut dendrite arms, whereby the grains are granulated and refined. However, Sn carries out such functions even more remarkably when Zn exists. In addition, gamma phases generated by the addition of Sn suppress the grain growth after the melt-solidification and thus contribute to the grain refinement. However, a high melting point Zr - Sn - Cu intermetallic compound, the melting point of which exceeds 1000°C, is likely to be formed when the amount of Sn exceeds 5%. Accordingly, it is preferable that the amount of Sn be smaller than that of Zn. Al improves the flowability of a molten alloy and prevents the oxidation / sulphurization loss of Zr, whereby Al contributes remarkably to the grain refinement in casting process when added with Zr and P. Furthermore, Al works to cut the dendrite arms, like Sn, so as to granulate the grains, and improves the strength, wear resistance or the like of the alloy.

Master alloys according to this invention can be manufactured by the following method.
Pure Cu is melted in non-oxidation atmosphere, and then Zn is added for deoxidation (First charge of Zn). In this case, the concentration of Zn should be in the range of 3 to 25% in consideration of the relationship with the temperature of the molten alloy as well as in consideration of the relationship with the vapor pressure of Zn. The temperature of the molten alloy is raised up to 1100 to 1200°C, and a predetermined amount of a commercial master alloy Cu - Zr (Zr occupies 10 to 60%) is charged. And, finally, a low melting point Zn is charged (Second charge of Zn). Subsidiary components such as B and Mg (active metals) are added at the same time as or after the second charge of Zn. In the case of Sn, Al, Mn, Si (to be added in the form of pure Si or Cu - 15 Si), and P (to be added in the form of Cu - 15 P), it is preferable that a predetermined amount be added after the first charge of Zn or at the same time as or after the second charge of Zn.
The above intermediate alloys are poured in the shape of a boat, a grain or the like, or manufactured by a continuous casting in the shape of a rod or a wire. Alternatively, the intermediate alloys are once manufactured into a large-sized casting, and then the large-sized casting is formed into wire, rod, plate, or thin plate by hot extrusion or hot rolling.

When such master alloys are charged into a melting furnace, a holding furnace, a tundish or the like simultaneously or continuously, a predetermined concentration of Zr can be secured in the molten copper alloy with the presence of a predetermined concentration of P.

### (Manufacturing a rod, a wire, a hollow bar, and a large-sized ingot by continuous casting)

Basically, components other than Zr are added within a predetermined composition range of an alloy. In consideration of raw material conditions (such as raw materials being contaminated with oil etc.), desulphurization and deoxidation additives such as Mg, Sn, Al, Mn, and Si are further added within the effective component range (or equal to or less than the concentration of impurities), and then desulphurization and deoxidation are carried out for confirmation. Generally, the melting furnace, gutter, tundish, distributor are coated with charcoal in order to block themselves from the air. Meanwhile, in the case of the grain-refining element P, it is preferable that the shortfall of P be replenished by charging Cu - P alloy (generally, P occupies 10 to 15%) into the melting furnace.

There are two methods for adding Zr, as described below. The concentration of Zr and what subsidiary components to be contained in a Zr master alloy are determined on the basis of the features of the alloy to be modified (melting point, additives or the like).
First of all, a master alloy is charged into the melting furnace in order to make the alloy contain the predetermined amount of Zr. And then, a casting such as ingot, billet etc. is cast. Meanwhile, it takes a long time to complete the whole casting process (semi-continuous casting), whereby the oxidation loss of Zr happens in the melting furnace or the like. In order to replenish the shortfall of Zr, master alloys in the form of several to 20 millimeter-large grains or wire or rod are further charged continuously or at regular intervals to the tundish and the distributor prior to the pouring. In this case, the melting point of the master alloy must be lower than the pouring temperature of the alloy. In the case where the master alloy can be melted completely in the tundish and the distributor within one minute without stirring, Zr can be precisely added by measuring the melting loss of Zr during the casting beforehand.

In the other method, after the deoxidation and desulphurization and the addition of P, the molten alloy is flowed into the tundish or the distributor, and, there, a master alloy is added in order to make the alloy contain a predetermined concentration of Zr. While casting continuously, master alloys in the form of several to 20 millimeter-large granular, wire or rod are continuously charged into the tundish or the distributor. When 50 ppm of Zr is required, a master alloy containing 5% of Zr occupies at most 1/1000 of the required amount of Zr, whereby no problem happens during the casting. In case of the addition of a master alloy to the tundish or the distributor, it is preferable that the amount of Zr corresponding to the loss amount, say 1 to 40% of extra Zr, be added.
Meanwhile, when the components are added continuously, the top priority is to melt the components quickly (the second priority is not to oxidize the components), whereby it is preferable that a master alloy contain 1 to 10% of Zr and have the concentration of Cu in the range of 50 to 65%. Furthermore, it is preferable that a master alloy contain elements that can lower the melting point of each alloy system. Meanwhile, when the components are added into the melting furnace, it is important to melt the components quickly, however, it is also important that Zr does not form oxidation and/or sulphurization in order to keep the loss of Zr minimum.

### (In the case of low-pressure casting, die casting, molten alloy forging (metal fittings for water supply, water meters or the like))

In the above-mentioned casting methods, a melting furnace is highly airtight, whereby it is common that raw materials are charged into the melting furnace little by little as may be necessary during the casting in order to replenish the reduced amount of molten alloy for the manufacturing of a casting. In addition, when all raw materials are charged simultaneously, the temperature of the melting furnace decreases, and thus the casting temperature also decreases, therefore, in general, raw materials are not charged simultaneously during operation time, but charged at the intervals of operations such as early in the morning, during lunch time, and late at night. That is, generally, a small amount of raw materials is charged to stabilize the molten temperature as much as possible. The above continuous operation can be carried out by, largely, two methods.

The first method is to charge raw materials containing no Zr and a master alloy in order to make the amount of Zr reach a predetermined value. In this case, the master alloy is prepared in a granular form, or by cutting the master alloy in the form of rod, wire, boat or the like into a certain length. In addition, process scrap and defective products in runners, which are sequentially generated, and which oxidation or sulphurization is rarely generated therein, are positively used in the continuous operation. In this case, the master alloy is added in consideration of the amount of Zr contained in the scraps. Meanwhile, when disposed products or the like are used as raw materials, the disposed products are used at the intervals between operations and, in this case, Zr master alloy is added after the molten alloy is oxidized and sulphurized sufficiently by Mg, Al or the like.
The other method is to charge ingots containing a predetermined amount of Zr at regular intervals (in consideration of the loss amount of Zr).

### (In the case of a batch-type casting such as sand casting etc.)

Since the components are melted simultaneously in a large melting furnace, basically, the process is identical to the above-mentioned process. What is different is that, while a continuous casting is adopted in the above-mentioned process, a batch-type casting is adopted in this case. In sand casting, it is normal that the molten alloy is ladled and then poured into a sand molding. The difference is that a sufficiently oxidized and sulphurized molten master alloy is charged into the melting furnace in case of continuous casting, while a master alloy is charged into the ladle in case of sand casting.

The casting methods of the invention are useful for preparing a copper alloy, wherein a small amount of Zr is added in the presence of P; firstly primary alpha phases are crystallized; secondarily peritectic or eutectic reaction occurs during solidification; and then the grains are refined. Specifically, such copper alloy includes Cu - Zn, Cu - Zn - Si, Cu - Zn -Sn, Cu - Zn - Al, Cu - Zn - Pb, Cu - Zn - Bi, Cu - Zn -Si - Mn, Cu - Zn -Si - Pb, Cu -Zn - Si - Sn, Cu - Zn - Si - Al, Cu - Zn - Sn - Pb, Cu - Zn - Sn - Bi, Cu - Zn - Sn - Al, Cu - Sn, Cu - Sn - Pb, Cu - Sn - Bi, Cu - Al, Cu - Al - Si, Cu - Si, Cu - Cr, Cu - Pb, Cu - P, and Cu - Te. Master alloys in Table 4 are used for each copper alloy after the composition ratios of the master alloys are adjusted in the above-mentioned ranges. Particularly, when such master alloys are used, it is required to prevent the loss of the master alloy (the loss of effective Zr contained in the master alloy) with cares on the followings: 1) the molten alloy is deoxidized and desulphurized beforehand and 2) the melting temperature and the casting temperature is in the appropriate range.

For the above-mentioned copper alloys, Zr is added in an amount of 5 ppm or more, preferably 20 to 500 ppm Zr, in the presence of P, preferably 0.01 to 0.35 mass% P.

Zr, like other additives, can refine the grains of a copper alloy slightly by itself; however, Zr can refine the grains remarkably in the presence of P. Even though Zr can refine the grains at the amount of 5 ppm or more, the grains can be refined remarkably when 10 ppm or more of Zr is added, and further remarkably when 20 ppm or more of Zr is added. Therefore, the amount of Zr should be 5 ppm or more, preferably 10 ppm or more, and more preferably 20 ppm or more. However, the minimum amount of Zr, at which the grains are refined by Zr in the presence of P, considerably depends on the composition of matrix. For example, for Cu - Sn alloy, Cu - Sn - Zn alloy, Cu - Sn - Zn - Pb alloy, Cu - Sn - Zn - Bi alloy, Cu - Si alloy, Cu - Si - Zn alloy, Cu - Zn alloy, Cu - Zn - (Bi, Pb) alloy, Cu - Al alloy, Cu - Zn - Al alloy, Cu - Zn - Al - Sn alloy, Cu - Zn - Al - Sn - (Bi, Pb) alloy and Cu - Zn - Al - (Bi, Pb) alloy, the grains are refined effectively even when the amount of Zr is 5 ppm. However, for the copper alloys having the composition close to pure Cu (for example, copper alloys satisfying [Zn] + 3 × [Sn] + 5 × [Si] + 3 × [Al] + 0.5 × [Bi] + 0.5 × [Pb] < 15), it is preferable that the amount of Zr should be 50 ppm or more in order to refine the grains effectively.

On the other hand, if the amount of Zr exceeds 0.3 mass%, the grain refining function of Zr is saturated regardless of the types or amounts of the other components. Meanwhile, since Zr has an extremely strong affinity to oxygen, when an alloy is melted in the air or scrap materials are used as raw materials, Zr is likely to become oxide or sulfide. Accordingly, if Zr is added excessively, the oxide or sulfide is included during the casting. In order to avoid such problem, it can be considered to melt and cast the alloy under vacuum or completely inactive gas atmosphere. However, in this case, the casting method will lose its general versatility and consequently, the casting cost rises drastically for a modified copper alloy, to which Zr is added only as a grain refining element. Considering the above problem, to modify a copper alloy according to this invention, the amount of Zr, which is not formed in the form of oxide or sulfide, should be 500 ppm or less, preferably 300 ppm or less, and optimally 200 ppm or less.

In addition, if the amount of Zr is in the above range, even when the modified copper alloy is melted in the air as a recycled material, the amount of zirconium oxide or zirconium sulfide generated during the melting is reduced, and robust modified copper alloy can be obtained. Furthermore, it is possible to easily transform the modified copper alloy into a copper alloy to be modified.

In addition, from a view point of casting products, it is preferable to add Zr, which is not oxidized or sulphurized, in the form of granular substance or thin plate-like substance, or as an intermediate alloy in such forms as granular or thin plate-like right before the pouring during the casting. That is, as described above, since Zr is an easily oxidized element, it is preferable to add Zr right before the pouring during the casting. However, in this case, since the melting point of Zr is higher than that of a copper alloy by 800 to 1000°C, it is preferable to add Zr as granular substance (grain diameter: about 2 to 50 mm), thin plate substance (thickness: about 1 to 10 mm) or); or a low melting point master alloy in the granular form or thin plate-like form having the melting point close to that of the copper alloy and containing a great amount of necessary elements.

Meanwhile, like Zr, P itself can refine the grains of a cast alloy slightly, however, P can refine the grains remarkably in the presence of Zr, or Zr and Si. That is, even though 100 ppm (0.01 mass%) or more of P can refine the grains, at least 300 ppm or more of P is required to refine the grains remarkably when no Si is added, however, only 200 ppm or more of P can refine the grains remarkably when Si is added. In addition, if 300 ppm or more of P is contained when Si is added, the grains can be refined further remarkably.

On the other hand, if the amount of P exceeds 0.35 mass%, the grain refining function of P is saturated. In order to effectively refine the grains without negative influence on the inherent features of an alloy in the casting method, in which P is added as a grain refining element, it is preferable that the amount of P be 0.25 mass% or less, more preferably 0.2 mass% or less, and optimally 0.15 mass%.

Meanwhile, it can be considered that an intermetallic compound of Zr and P may play a role in the grain refinement process, and the amount ratio of P/Zr should satisfy 0.5 < P/Zr < 150, preferably 1 < P/Zr < 50, and optically 1.2 < P/Zr < 25. By limiting the amount ratio of P/Zr in the above range, the primary alpha phases can be crystallized during the solidification, and then beta phases can be crystallized by peritectic or eutectic reaction. Accordingly, the grains can be refined.

Since the invention relates to the methods of refining grains during the casting process, it is possible to improve the hot workability of a copper alloy, and thus to perform the processing work such as rolling, forging, extruding, drawing or the like satisfactorily after casting.

According to the invention, the casting methods (wherein casting products, ingot, slab or the like are obtained by 1) sand casting, 2) permanent mold casting, 3) low-pressure casting, 4) continuous casting, 5) die casting, 6) squeeze, 7) lost wax casting 8) semi-solid (semi-solid metal solidifying method), 9) molten alloy forging) can achieve the strength improvement (comparing to a copper alloy to be modified, the strength and the proof strength are improved by 10 to 20% or more, and the elongation or the like is improved to the same or more degree), brittleness reduction, wall thickness reduction, weight lightening, toughness improvement, impact characteristic improvement, ductility improvement, casting defect (porosity, shrinkage cavity, hole, crack- or the like) reduction or the like of copper alloy casting products. Therefore, it is possible to obtain high quality casting products including complex shaped products, extremely large-sized and small-sized products.

In addition, according to the invention, since the casting methods provide castings (casting products), particularly produced by permanent mold casting or continuous casting, which have the same degree of grain size and strength as those of hot extruding material or drawing material of a copper alloy to be modified, the castings according to the present invention can replace the extruding material and the drawing material (or forging material made of the hot extruding material or the drawing material). The castings according to the present invention need not to be subjected to the working processes such as extrusion or the like, whereby the manufacturing cost can be reduced considerably, and the energy can be saved.

In order to effectively refine the grains during the melt-solidification in any casting methods, it is preferable that the primary crystal be alpha phases during the melt-solidification, and that beta phases occupy 95% or less in the total phase structure right after the melt-solidification and also 50% or less at the room temperature after the melt-solidification. It is more preferable that beta phases occupy 20% or less in the total phase structure at the room temperature and beta phases be transformed into alpha, kappa, gamma, delta, and mu-phases. Furthermore, if an adequate amount of predetermined phases (one to three phases among beta, kappa, gamma, and delta phases) exist at a high temperature right after the melt-solidification, the beta, kappa and gamma phases suppress the growth of alpha grains and thus the grains are effectively refined. Therefore, it is preferable that beta, kappa, gamma and delta phases occupy 5 to 95% of the surface ratio (total) in the phase structure at the high temperature right after the melt-solidification. It is also preferable that the phase diagram include one to four phases selected from alpha, beta, kappa, gamma, delta or mu phases at the room temperature after melt-solidification. Meanwhile, kappa, gamma, delta and mu phases existing at the room temperature after the melt-solidification have no adverse influence on grain refinement. In addition, in the case of a copper alloy containing Zn and Si, the above phases contribute to the grain refinement, and, particularly, the grains are refined remarkably when kappa and/or gamma phases exist abundantly. Meanwhile, when a lot of beta phase exists (for example, when beta phase occupies more than 10% of the surface ratio in the phase diagram at the room temperature), even though the corrosion resistance or ductility of the castings (permanent mold casting products or the like) may deteriorate, such problems can be solved by conducting an adequate heat treatment on the castings (for example, heat treatment at 400 to 600°C for 10 minutes to 4 hours). That is, heat treatment can remove or divide beta phases. The effect of the heat treatment to remove and divide beta phases becomes more remarkable as the grain size becomes smaller.

In addition, in order to drastically refine the grains in both macro-structure and micro-structure, it is preferable that the forms of solid phases during the melt-solidification, or the grains or alpha phases at the room temperature after the melt-solidification be circular or substantially circular when observed two-dimensionally, which is formed by cutting the dendrite arms. That is, it is preferable that the two dimensional forms be non-dendritic, circular, oval, cross-like, needle-like or polygonal. Particularly, if the solid phases have the dendrite arms spreading like a net within the castings (castings including ingot, slab, die casting or the like, semi-solid metal forging products or the like), the grains of which are strongly desired to be substantially circular and small, otherwise, flowability of the molten alloy deteriorates and substantial defects such as porosity, shrinkage cavity, blowhole, casting crack or the like occur. However, if the two dimensional forms are circular or substantially circular and then the solid phases are granulated, flowability to every corner is notably improved and thus high quality casting products can be obtained. The improvement of flowability (molten alloy flowability) is profitable and practically effective in the semi-solid metal casting method or semi-solid metal forging method performed in a semi-solid metal state (solid phase + liquid phase). For example, it is not required to perform a grain refining treatment (for example, steering, electromagnetic induction agitation, hot working (hot extrusion, drawing or the like)) as a pretreatment on materials used in the semi-solid metal forging method (For this reason, it becomes preferable for, particularly, thixo-casting). Furthermore, when the grains are small and substantially circular, a casting having such grains shows a strong resistance against the crack caused by thermal distortion or the like during and right after the melt-solidification. In addition, even when used as ingot, such castings have a great deformability. Therefore, materials difficult to be hot worked can also be easily obtained without crack.

Generally, except when a casing is rapidly solidified or under special techniques such as the above electromagnetic induction agitation or the like, the grain size of the casting (melt-solidified copper alloy) is larger than that of the material produced by post-casting treatment such as rolling or the like applying distortion energy and is larger ten times or more thereof. That is, grains should be refined as long as a great amount of energy is consumed. Therefore, from a technical viewpoint, it is inadequate to treat 'castings with the grains refined during the melt-solidification', and 'castings with the grains refined by post-casting treatment like methods (e) and (f)' in the same category. However, as understood from the following examples, comparing to a copper alloy with the refined grains by extruding, drawing or rolling, the grain size of a modified copper alloy of the invention, the grains of which are refined during the casting process, is almost equal, and the mechanical strength is also almost equal or higher. It deserves attention that a casting, produced simply by melting and solidifying a predetermined composition, has the almost same mechanical strength as that of a casting produced by consuming a great amount of energy through rolling or the like.

Furthermore, in comparison with a copper alloy to be modified, the proof strength of casting products of a modified copper alloy(0.2% proof strength of ingot or the like after the melt-solidification) is improved by 10% or more (preferably 20% or more, more preferably 30% or more, and optically 40% or more) through the grain refinement, when both alloys are cast under the identical condition except for the grains being refined (in the modified copper alloy) or not (in the copper alloy to be modified).

### (Manufacturing master alloys)

Master alloys disclosed in Tables 1 to 3 are manufactured by the above-mentioned methods of manufacturing a master alloy.

In the following Table 1, 75 ppm Zr + 0.06% P is added to Alloy 1: 76Cu - 3Si - 21Zn alloy, and the optimal amount of Zr (which is not in the form of oxide and sulfide) is defined as 25 to 75 ppm.

In the following Table 2, 100 ppm Zr + 0.06% P is added to Alloy 2: 73Cu - 25.5Zn - 1.5Sn alloy, and the optimal amount of Zr (which is not in the form of oxide and sulfide) is defined as 40 to 100 ppm.

In the following Table 3, 200 ppm Zr + 0.06% P is added to Alloy 3: 90Cu + 10Sn alloy, and the optimal amount of Zr (which is not in the form of oxide and sulfide) is defined as 120 to 200 ppm.

### EXAMPLE 1

Electrolytic Cu, electrolytic Zn, electrolytic Sn, and Cu - 15% Si alloy are melted in the descending order of melting point, which is Cu, Cu-15% Si alloy, Zn and Sn, and then Cu - 15P is added so that the total mass reaches about 3 kilograms. The temperature of the final molten alloy is set at approximately 100°C above the liquidus line temperature of each alloy (that is, 970°C for Alloy 1, 1040°C for Alloy 2, and 1120°C for Alloy 3). After 5-minute holding, Zr master alloys disclosed in Tables 1 to 3 are added so that a predetermined amount of Zr can be contained at the final stage. After 10-second stirring by a graphite rod, the alloy is held for one minute, and then again, is stirred by the graphite rod for about 5 seconds. After that, the molten alloy is poured into a φ 40 × 250(l) or 35t × 65w × 200(1) sized metal mold.
Meanwhile, as a comparative example, a predetermined amount of Cu - 35Zr and Cu - 50Zr alloys is added.

Furthermore, the holding time is extended for some alloys.
Each of the master alloys is cut into a cube having sides of about 5 mm, and then is cut again to contain a predetermined Zr amount.

The pouring temperature is, generally, in the range of 30 to 150°C above the liquidus line temperature. If the pouring temperature is too high, casting defects such as crack or the like are likely to occur. The melting temperature is, generally, 50°C above the pouring temperature in consideration of the temperature decrease in a runner or the like. An unreasonable rise of temperature results in the waste of energy.

40 mm-long pieces are cut off from the top and bottom of the complete castings, and then the surfaces thereof are grinded. After that, the macro-structure is developed by nitric acid, and then the real scale and 7.5 times magnified grain size are measured by a magnifying glass according to JIS comparative method.

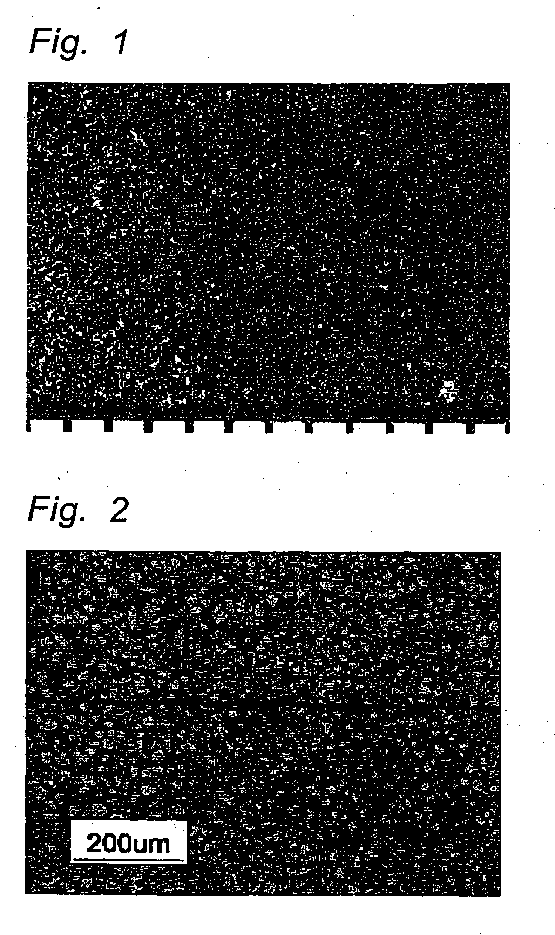

Fig. 1 is a view showing the macro-structure of 76Cu - 3Si - 21Zn casting that is cast by using a master alloy of Sample No. 1 (62Cu - 3Zr - 35Zn) in Table 1, the surface of which is treated by nitric acid and then observed by a magnifying glass of 7.5 times magnification. Fig. 2 is a view showing the micro-structure of 76Cu - 3Si - 21Zn casting that is cast by using the master alloy of Sample No. 1 in Table 1, the surface of which is treated by hydrogen peroxide and ammonia and then observed by a metallurgical microscope. From Fig. 2, it can be understood that, in the casting alloy, the grain size is 50 µm or less and, accordingly, the grains are refined.

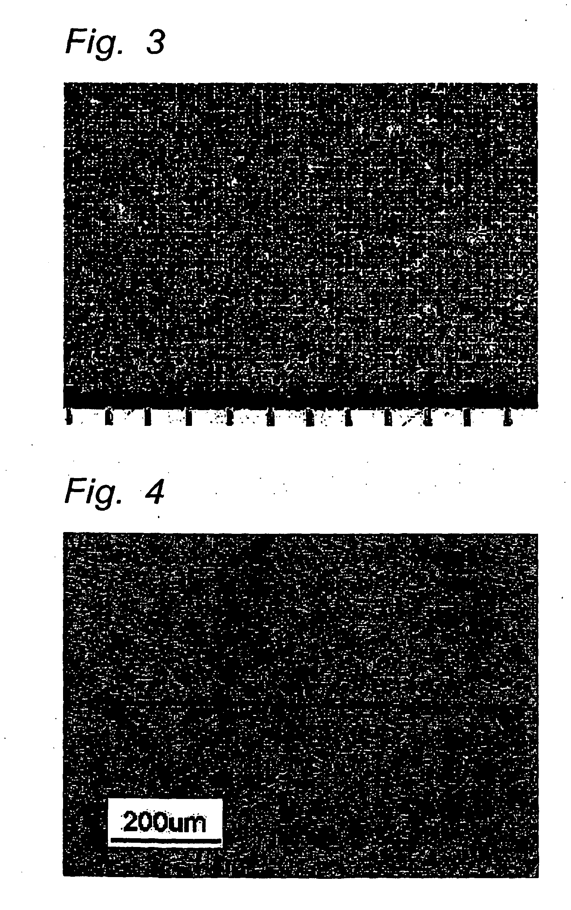

Furthermore, Fig. 3 is a view showing the macro-structure of 76Cu - 3Si - 21Zn casting that is cast by using a master alloy of Sample No. 13 (50Cu - 50Zr) in Table 1, the surface of which is treated by nitric acid and then observed by a magnifying glass of 7.5 times magnification. Fig. 4 is a view showing the micro-structure of 76Cu - 3Si - 21Zn casting that is cast by using the master alloy of Sample No. 13 in Table 1, the surface of which is treated by hydrogen peroxide and ammonia and then observed by a metallurgical microscope. The grain size in the casting products manufactured by using this master alloy is 150 µm.

**[Table 1]**

| Master alloy (75 ppm Zr + 0.06% P) is added to 76Cu - 3Si - 21Zn alloy | | | | | | | | |
|---|---|---|---|---|---|---|---|---|
| No. | Type of master alloys (%) | | | | | Casting result | | Remark |
| | Cu | Zr | Zn | Others | | Zr | grain size µm | |
| 1 | 62 | 3 | 35 | | 0 | 69 | 50 µm or less | |
| 2 | 61 | 0.9 | 38.1 | | 0 | 71 | 50 µm or less | |
| 3 | 58 | 6 | 36 | | 0 | 68 | 50 µm or less | |
| 4 | 76 | 3 | 21 | | 0 | 67 | 50 µm or less | |
| 5 | 44 | 31 | 25 | | 0 | 60 | 50 µm or less | |
| 6 | 55 | 12 | 33 | | 0 | 65 | 50 µm or less | |
| 7 | 60 | 4 | 35.5 | Mg | 0.5 | 71 | 50 µm or less | |
| 8 | 58 | 6 | 34 | Al | 2 | 70 | 50 µm or less | |
| 9 | 60 | 4 | 35.4 | Si | 0.6 | 71 | 50 µm or less | |
| 10 | 60 | 4 | 35.7 | B | 0.3 | 71 | 50 µm or less | |
| 11 | 57 | 6 | 35 | Mn | 2 | 68 | 50 µm or less | |
| 12 | 55 | 4 | 40 | P | 1 | 70 | 50 µm or less | |
| 13 | 50 | 50 | 0 | | 0 | 12 | 200 | Part of master alloy not melted |
| 14 | 50 | 50 | 0 | | 0 | 32 | 150 | Bottom: 100 µm, Top: 200 µm, Holding time: 3 min. extended |
| 15 | 65 | 35 | 0 | | 0 | 15 | 150 | Part of master alloy not melted |
| 16 | 65 | 35 | 0 | | 0 | 43 | 125 µm or less | Bottom: 50 µm, Top: 200 µm, Holding time: 3 min. extended |

**[Table 2]**

| Master alloy (100 ppm Zr + 0.06% P) is added to 73Cu - 25.5Zn - 1.5Sn alloy | | | | | | | | |
|---|---|---|---|---|---|---|---|---|
| No. | Type of master alloys (%) | | | | | Casting result | | Remark |
| | Cu | Zr | Zn | Others | | Zr | grain size µm | |
| 17 | 62 | 3 | 35 | | 0 | 90 | 50 µm or less | |
| 18 | 61 | 0.9 | 38.1 | | 0 | 89 | 50 µm or less | |
| 19 | 58 | 6 | 36 | | 0 | 87 | 50 µm or less | |
| 20 | 76 | 3 | 21 | | 0 | 86 | 50 µm or less | |
| 21 | 44 | 31 | 25 | | 0 | 76 | 50 µm or less | |
| 22 | 55 | 12 | 33 | | 0 | 82 | 50 µm or less | |
| 23 | 60 | 4 | 35.5 | Mg | 0.5 | 90 | 50 µm or less | |
| 24 | 58 | 6 | 34 | Al | 2 | 92 | 50 µm or less | |
| 25 | 60 | 4 | 33 | Sn | 3 | 89 | 50 µm or less | |
| 26 | 57 | 6 | 35 | Mn | 2 | 90 | 50 µm or less | |
| 27 | 55 | 4 | 40 | P | 1 | 91 | 50 µm or less | |
| 28 | 50 | 50 | 0 | | 0 | 27 | 300 | Part of master alloy not melted |
| 29 | 50 | 50 | 0 | | 0 | 55 | 1000 | Bottom: 500 µm, Top: 150 µm, Holding time: 3 min. extended |
| 30 | 50 | 50 | 0 | | 0 | 53 | 275 | Bottom: 150 µm, Top: 400 µm Holding time: 3 min. extended, Second experiment |
| 31 | 65 | 35 | 0 | | 0 | 57 | 175 | Bottom: 100 µm, Top: 250 µm |

**[Table 3]**

| Master alloy (200 ppm Zr + 0.06% P) is added to 90Cu - 10Sn alloy | | | | | | | | |
|---|---|---|---|---|---|---|---|---|
| No. | Type of master alloys (%) | | | | | Casting result | | Remark |
| | Cu | Zr | Zn | Others | | Zr | grain size µm | |
| 32 | 62 | 3 | 35 | | 0 | 178 | 50 µm or less | |
| 33 | 61 | 0.9 | 38.1 | | 0 | 182 | 50 µm or less | |
| 34 | 58 | 6 | 36 | | 0 | 173 | 50 µm or less | |
| 35 | 76 | 3 | 21 | | 0 | 176 | 50 µm or less | |
| 36 | 44 | 31 | 25 | | 0 | 157 | 50 µm or less | |
| 37 | 55 | 12 | 33 | | 0 | 166 | 50 µm or less | |
| 38 | 60 | 4 | 35.5 | Mg | 0.5 | 176 | 50 µm or less | |
| 39 | 58 | 6 | 34 | Al | 2 | 180 | 50 µm or less | |
| 40 | 60 | 4 | 33 | Sn | 3 | 179 | 50 µm or less | |
| 41 | 57 | 6 | 35 | Mn | 2 | 178 | 50 µm or less | |
| 42 | 55 | 4 | 40 | P | 1 | 181 | 50 µm or less | |
| 43 | 50 | 50 | 0 | | 0 | 75 | 400 | Part of master alloy not melted |
| 44 | 50 | 50 | 0 | | 0 | 118 | 250 | Bottom: 100 µm, Top: 400 µm Second experiment |
| 45 | 65 | 35 | 0 | | 0 | 115 | 175 | Bottom: 100 µm, Top: 250 µm |

### EXAMPLE 2

For each alloy system in Table 4, a specific composition of an alloy is adjusted to make the copper alloy meet 60 < Cu - 3.5Si - 1.8Al - 0.5X + 0.5Y + Mn < 90 (wherein X is Sn, Sb, As, Mg; Y is Pb, Bi, Se, Te, Cr; preferably in the range of 62 to 71, and more preferably in the range of 63 to 67). In casting, typical master alloys indicated in the rightmost column are adjusted in the range of the invention and then added. Only examples with master alloys as defined in claim 1 belong to the invention. In the same way as Example 1, 40mm-long pieces are cut off from the top and bottom of the casting and then the surfaces thereof are grinded. After that, the macro-structure is developed by nitric acid, and then the real scale and 7.5 times magnified grain size are measured by a magnifying glass according to JIS comparative method. In any cases, the grain size is 50 µm or less.

**[Table 4]**

| Typical composition of alloys to be modified (grain refined) | | | | | | | | | | | | |
|---|---|---|---|---|---|---|---|---|---|---|---|---|
| Alloy system | Cu | Zn | Si | Sn | Al | Pb | Bi | Mn | Cr | P | Te | Typical master alloy |
| | | | | | | | | | | | | Cu-Zn-Zr, |
| Cu-Zn | 70 | remainder | | | | | | | | | | Cu-Zn-Zr-P, |
| | | | | | | | | | | | | Cu-Zn-Zr-B |
| | | | | | | | | | | | | Cu-Zn-Zr, |
| Cu-Zn-Si | 76 | remainder | 3 | | | | | | | | | Cu-Zn-Zr-P, |
| | | | | | | | | | | | | Cu-Zn-Zr-Si |
| Cu-Zn-Si **2 | 79 | remainder | 3.8 | | | | | | | | | Same as above |
| | | | | | | | | | | | | Cu-Zn-Zr, |
| Cu-Zn-Sn | 69.5 | remainder | | 1.2 | | | | | | | | Cu-Zn-Zr-P, |
| | | | | | | | | | | | | Cu-Zn-Zr-Sn |
| Cu-Zn-Sn **2 | 78 | remainder | | 2.5 | | | | | | | | Same as above |
| | | | | | | | | | | | | Cu-Zn-Zr, |
| Cu-Zn-Al | 77 | remainder | | | 2 | | | | | | | Cu-Zn-Zr-P, |
| | | | | | | | | | | | | Cu-Zn-Zr-Al |
| | | | | | | | | | | | | Cu-Zn-Zr, |
| Cu-Zn-Pb | 63 | remainder | | | | 1 | | | | | | Cu-Zn-Zr-P, |
| | | | | | | | | | | | | Cu-Zn-Zr-Mg |
| | | | | | | | | | | | | Cu-Zn-Zr, |
| Cu-Zn-Bi | 63 | remainder | | | | | 1 | | | | | Cu-Zn-Zr-P, |
| | | | | | | | | | | | | Cu-Zn-Zr-Mg |
| | | | | | | | | | | | | Cu-Zn-Zr, Cu-Zn-Zr-P, Cu-Zn-Zr-Mn |
| Cu-Zn-Si-Mn | 73 | remainder | 4 | | | | | 3 | | | | |
| | | | | | | | | | | | | |
| Cu-Zn-Si-Mn **2 | 64 | remainder | 1 | | | | | 3 | | | | Same as above |
| | | | | | | | | | | | | Cu-Zn-Zr, |
| Cu-Zn-Si-Pb | 76 | remainder | 3 | | | 0.1 | | | | | | Cu-Zn-Zr-P, |
| | | | | | | | | | | | | Cu-Zn-Zr-B |
| | | | | | | | | | | | | Cu-Zn-Zr, |
| Cu-Zn-Si-Sn | 77 | remainder | 3 | 0.4 | | | | | | | | Cu-Zn-Zr-P, |
| | | | | | | | | | | | | Cu-Zn-Zr-Si |
| Cu-Zn-Si-Sn **2 | 75 | remainder | 1.5 | 0.5 | | | | | | | | Same as above |
| | | | | | | | | | | | | Cu-Zn-Zr, |
| Cu-Zn-Si-Al | 77 | remainder | 3 | | 0.5 | | | | | | | Cu-Zn-Zr-P, |
| | | | | | | | | | | | | Cu-Zn-Zr-Al |

**[Table 5]**

| | | | | | | | | | | | | |
|---|---|---|---|---|---|---|---|---|---|---|---|---|
| | | | | | | | | | | | | Cu-Zn-Zr, Cu-Zn-Zr-P, Cu-Zn-Zr-Sn |
| Cu-Zn-Sn-Pb | 64 | remainder | | 1.5 | | 1 | | | | | | |
| | | | | | | | | | | | | |
| Cu-Zn-Sn-Pb **2 | 84 | remainder | | 5 | | 4 | | | | | | Same as above |
| | | | | | | | | | | | | Cu-Zn-Zr, |
| Cu-Zn-Sn-Bi | 82 | remainder | | 5 | | | 2 | | | | | Cu-Zn-Zr-P, |
| | | | | | | | | | | | | Cu-Zn-Zr-Sn |
| Cu-Zn-Sn-Bi **2 | 63 | remainder | | 1 | | | 1 | | | | | Same as above |
| | | | | | | | | | | | | Cu-Zn-Zr, |
| Cu-Zn-Sn-Al | 74 | remainder | | 1.5 | 0.5 | | | | | | | Cu-Zn-Zr-P, |
| | | | | | | | | | | | | Cu-Zn-Zr-Al |
| | | | | | | | | | | | | Cu(high)-Zn-Zr, |
| Cu-Sn | 90 | | | 10 | | | | | | | | Cu-Zn-Zr-P, |
| | | | | | | | | | | | | Cu(high)-Zn-Zr-Sn |
| | | | | | | | | | | | | Cu(high)-Zn-Zr, |
| Cu-Sn-Pb | 83 | | | 9 | | 8 | | | | | | Cu-Zn-Zr-P, |
| | | | | | | | | | | | | Cu(high)-Zn-Zr-Sn |
| | | | | | | | | | | | | Cu(high)-Zn-Zr, |
| Cu-Sn-Bi | 89 | | | 6 | | | 5 | | | | | Cu-Zn-Zr-P, |
| | | | | | | | | | | | | Cu(high)-Zn-Zr-Sn |
| | | | | | | | | | | | | Cu(high)-Zn-Zr, |
| Cu-Al | 92 | | | | 8 | | | | | | | Cu-Zn-Zr-P, |
| | | | | | | | | | | | | Cu-Zn-Zr-Al |
| | | | | | | | | | | | | Cu(high)-Zn-Zr, |
| Cu-Al-Si | 93 | | 2 | | 5 | | | | | | | Cu-Zn-Zr-P, |
| | | | | | | | | | | | | Cu-Zn-Zr-Al |
| | | | | | | | | | | | | Cu(high)-Zn-Zr, |
| Cu-Si | 97 | | 3 | | | | | | | | | Cu-Zn-Zr-P, |
| | | | | | | | | | | | | Cu-Zn-Zr-Si |
| | | | | | | | | | | | | Cu(high)-Zn-Zr, |
| Cu-Cr | 99 | | | | | | | | 1 | | | Cu-Zn-Zr-P, |
| | | | | | | | | | | | | Cu-Zn-Zr-Mg |
| | | | | | | | | | | | | Cu(high)-Zn-Zr, |
| Cu-P | 99.8 | | | | | | | | | 0.2 | | Cu-Zn-Zr-P, |
| | | | | | | | | | | | | Cu-Zn-Zr-Mg |
| | | | | | | | | | | | | Cu(high)-Zn-Zr, |
| Cu-Pb | 99 | | | | | 1 | | | | | | Cu-Zn-Zr-P, |
| | | | | | | | | | | | | Cu-Zn-Zr-Mg |
| | | | | | | | | | | | | Cu(high)-Zn-Zr, |
| Cu-Te | 99.3 | | | | | | | | | | 0.7 | Cu-Zn-Zr-P, |
| | | | | | | | | | | | | Cu-Zn-Zr-Mg |

| | | | | | | | | | | | | |
|---|---|---|---|---|---|---|---|---|---|---|---|---|
| ** means a case that there are two typical compositions. | | | | | | | | | | | | |

### Industrial Applicability

According to the methods of the invention for melting and solidifying a copper alloy to be modified, grains of modified copper alloy can be refined in continuous casting method, semi-solid metal casting method, sand casting method, permanent mold casting method, low-pressure casting method, die casting, lost wax, up casting, squeeze, centrifugal casting method, welding, lining, overlaying or build-up spraying.

## Claims

1. Use of a master alloy for casting a modified copper alloy, wherein the master alloy is a Cu-Zn-Zr alloy comprising
Cu: 40 to 80%, Zr: 0.5 to 35% and optionally at least one element selected from the group consisting of Mg: 0.01 to 1%, Al: 0.01 to 5%, Sn: 0.1 to 5%, B: 0.01 to 0.5%, Mn: 0.01 to 5% and Si: 0.01 to 1%, and the balance of Zn, wherein the amount of Zn is larger than the amount of Zr,
or a Cu-Zn-Zr-P alloy comprising
Cu: 40 to 80%, Zr: 0.5 to 35%, P: 0.01 to 3% and optionally at least one element selected from the group consisting of Mg: 0.01 to 1%, Al: 0.01 to 5%, Sn: 0.1 to 5%, B: 0.01 to 0.5%, Mn: 0.01 to 5% and Si: 0.01 to 1%, and the balance of Zn, wherein the amount of Zn is larger than the amount of Zr.

2. The use according to claim 1,
wherein said Cu occupies 50 to 65%, and said Zr occupies 1 to 10%.

3. The use according to claim 1 or 2,
wherein said master alloy is an ingot formed in the shape of a boat, continuous casting material formed in the shape of a rod or wire, or hot extrusion material formed in the shape of a rod or wire.

4. A method of casting a modified copper alloy containing Zr and P from a molten copper alloy, which method comprises:
providing a molten copper alloy;
adding at least Zr in the form of a Cu-Zn-Zr or Cu-Zn-Zr-P master alloy as defined in claim 1 to said molten copper alloy
and casting said molten copper alloy,
wherein a concentration of the metal Zr in the molten alloy is in a range of 5 ppm or more, preferably 20 to 500 ppm in a presence of P, when the molten copper alloy begins to solidify.

5. The method according to claim 4,
wherein an amount ratio of P to Zr in said molten copper alloy satisfies 0.5 < P/Zr < 150, preferably 1 < P/Zr < 50, and more preferably 1.2 < P/Zr < 25.

6. The method according to claim 4,
wherein primary alpha phases begin to be crystallized during solidification.

7. The method according to claim 6,
wherein beta phases are crystallized by peritectic or eutectic reactions.

8. The method according to claim 6,
wherein kappa, gamma, delta and/or mu phases are precipitated in an alpha phase matrix by a solid phase reaction.

9. The method according to claim 4,
wherein the copper alloy to be modified is one selected from the group consisting of Cu - Zn, Cu - Zn - Si, Cu - Zn - Sn, Cu - Zn - Al, Cu - Zn - Bi, Cu - Zn - Pb, Cu - Zn - Si - Mn, Cu - Zn - Si - Pb, Cu - Zn - Si - Sn, Cu - Zn - Si - Al, Cu - Zn - Sn - Pb, Cu - Zn - Sn - Bi, Cu - Zn - Sn - Al, Cu - Sn, Cu - Sn - Pb, Cu - Sn - Bi, Cu - Al, Cu - Al - Si, Cu - Si, Cu - Cr, Cu - Pb, Cu - P, and Cu - Te.

10. The method according to claim 9,
wherein said copper alloy to be modified satisfies 60 < Cu - 3.5Si - 1.8Al - 0.5X + 0.5Y + Mn < 90 where X is Sn, Sb, As or Mg and Y is Pb, Bi, Se, Te or Cr.

## Patentansprüche

1. Verwendung einer Vorlegierung zum Gießen einer modifizierten Kupferlegierung, wobei die Vorlegierung eine Cu-Zn-Zr-Legierung, umfassend
Cu: 40 bis 80 %, Zr: 0,5 bis 35 % und wahlweise mindestens ein Element, ausgewählt aus der Gruppe, bestehend aus Mg: 0,01 bis 1 %, Al: 0,01 bis 5 %, Sn: 0,1 bis 5 %, B: 0,01 bis 0,5 %, Mn: 0,01 bis 5 % und Si: 0,01 bis 1 % und einem Rest aus Zn, wobei die Menge an Zn größer als die Menge an Zr ist,
oder eine Cu-Zn-Zr-P-Legierung ist, umfassend
Cu: 40 bis 80 %, Zr: 0,5 bis 35 %, P: 0,01 bis 3 % und wahlweise mindestens ein Element, ausgewählt aus der Gruppe, bestehend aus Mg: 0,01 bis 1 %, Al: 0,01 bis 5 %, Sn: 0,1 bis 5 %, B: 0,01 bis 0,5 %, Mn: 0,01 bis 5 % und Si: 0,01 bis 1 % und einem Rest aus Zn, wobei die Menge an Zn größer ist als die Menge an Zr.

2. Verwendung gemäß Anspruch 1,
wobei das Cu 50 bis 65 % einnimmt und das Zr 1 bis 10 % einnimmt.

3. Verwendung gemäß Anspruch 1 oder 2,
wobei die Vorlegierung eine in Boot-Form gebildete Kokille, ein in Form eines Stabs oder Drahts gebildeter kontinuierlicher Gusswerkstoff oder ein in Form eines Stabs oder Drahts geformtes heißes Extrusionsmaterial ist.

4. Verfahren zum Gießen einer modifizierten Kupferlegierung, die Zr und P enthält aus einer geschmolzenen Kupferlegierung, wobei das Verfahren folgendes umfasst:
Bereitstellen einer geschmolzenen Kupferlegierung;
Hinzufügen von mindestens Zr in Form einer Cu-Zn-Zr oder Cu-Zn-Zr-P-Vorlegierung, wie in Anspruch 1 definiert, zu der geschmolzenen Kupferlegierung
und Gießen der geschmolzenen Kupferlegierung,
wobei die Konzentration des Metalls Zr in der geschmolzenen Legierung im Bereich von 5 ppm oder mehr, vorzugsweise 20 bis 500 ppm, in Gegenwart von P vorliegt, wenn die geschmolzene Kupferlegierung beginnt, sich zu verfestigen.

5. Verfahren gemäß Anspruch 4,
worin das Mengenverhältnis von P zu Zr in der geschmolzenen Kupferlegierung 0,5 < P/Zr < 150, vorzugsweise 1 < P/Zr < 50 und besonders bevorzugt 1,2 < P/Zr < 25 erfüllt.

6. Verfahren gemäß Anspruch 4,
worin die primären Alpha-Phasen während der Verfestigung zu kristallisieren beginnen.

7. Verfahren gemäß Anspruch 6,
worin die Beta-Phasen durch peritektische oder eutektische Reaktionen kristallisiert werden.

8. Verfahren gemäß Anspruch 6,
worin Kappa-, Gamma-, Delta- und/oder mu-Phasen mittels einer Festphasenreaktion in einer Alpha-Phasenmatrix abgeschieden werden.

9. Verfahren gemäß Anspruch 4,
worin die zu modifizierende Kupferlegierung eine ist, die aus der Gruppe ausgewählt wird, bestehend aus Cu - Zn, Cu - Zn - Si, Cu - Zn - Sn, Cu - Zn - Al, Cu - Zn - Bi, Cu - Zn - Pb, Cu - Zn - Si - Mn, Cu - Zn - Si - Pb, Cu - Zn - Si - Sn, Cu - Zn - Si - Al, Cu - Zn - Sn - Pb, Cu - Zn - SN - Bi, Cu - Zn - Sn - Al, CU - Sn, CU - Sn - Pb, Cu - Sn - Bi, Cu - Al, Cu - Al - Si, Cu - Si, Cu - Cr, Cu - Pb, Cu - P und Cu - Te.

10. Verfahren gemäß Anspruch 9,
worin die zu modifizierende Kupferlegierung 60 < Cu - 3,5Si - 1,8Al - 0,5X + 0,5Y + Mn < 90 erfüllt, worin X Sn, Sb, As oder Mg ist und Y Pb, Bi, Se, Te oder Cr ist.

## Revendications

1. Utilisation d'un alliage mère pour couler un alliage de cuivre modifié, dans laquelle l'alliage mère est un alliage Cu-Zn-Zr comprenant
Cu : 40 à 80 %, Zr : 0,5 à 35 % et éventuellement au moins un élément choisi dans le groupe constitué par Mg : 0,01 à 1 %, Al : 0,01 à 5 %, Sn : 0,1 à 5 %, B : 0,01 à 0,5 %, Mn : 0,01 à 5 % et Si : 0,1 à 1 %, et le reste de Zn, où la quantité de Zn est plus grande que la quantité de Zr,
ou un alliage Cu-Zn-Zr-P comprenant
Cu : 40 à 80 %, Zr : 0,5 à 35 %, P : 0,01 à 3 % et éventuellement au moins un élément choisi dans le groupe constitué par Mg : 0,01 à 1 %, Al : 0,01 à 5 %, Sen : 0,1 à 5 %, B : 0,01 à 0,5 %, Mn : 0,01 à 5 % et Si : 0,01 à 1 %, et le reste de Zn, où la quantité de Zn est plus grande que la quantité de Zr.

2. Utilisation selon la revendication 1, dans laquelle ledit Cu représente 50 à 65 % et ledit Zr représente 1 à 10 %.

3. Utilisation selon la revendication 1 ou 2, dans laquelle ledit alliage mère est un lingot façonné sous la forme d'une nacelle, un matériau de coulée continue façonné sous la forme d'une barre ou d'un fil, ou un matériau d'extrusion à chaud façonné sous la forme d'une barre ou d'un fil.

4. Procédé de coulée d'un alliage de cuivre modifié contenant Zr et P à partir d'un alliage de cuivre fondu, lequel procédé comprend :
la fourniture d'un alliage de cuivre fondu ;
l'addition d'au moins Zr sous la forme d'un alliage mère Cu-Zn-Zr ou Cu-Zn-Zr-P tel que défini dans la revendication 1 audit alliage de cuivre fondu
et la coulée dudit alliage de cuivre fondu,
dans lequel une concentration du Zr métallique dans l'alliage fondu est dans une gamme de 5 ppm ou plus, de préférence 20 à 500 ppm, en présence de P, lorsque l'alliage de cuivre fondu commence à solidifier.

5. Procédé selon la revendication 4, dans lequel un rapport en quantité de P sur Zr dans ledit alliage de cuivre fondu satisfait à 0,5 < P/Zr < 150, de préférence 1 < P/Zr < 50 et de préférence encore 1,2 < P/Zr < 25.

6. Procédé selon la revendication 4, dans lequel des phases alpha primaires commencent à cristalliser pendant la solidification.

7. Procédé selon la revendication 6, dans lequel des phases bêta sont cristallisées par des réactions péritectiques ou eutectiques.

8. Procédé selon la revendication 6, dans lequel des phases kappa, gamma, delta et/ou mu sont précipitées dans une matrice de phase alpha par une réaction en phase solide.

9. Procédé selon la revendication 4, dans lequel l'alliage de cuivre à modifier est un alliage choisi dans le groupe constitué par Cu - Zn, Cu - Zn - Si, Cu - Zn - Sn, Cu - Zn - Al, Cu - Zn - Bi, Cu - Zn - Pb, Cu - Zn - Si - Mn, Cu - Zn - Si - Pb, Cu - Zn - Si - Sn, Cu - Zn - Si - Al, Cu - Zn - Sn - Pb, Cu - zn - Sn - Bi, Cu - Zn - Sn - Al, Cu - Sn, Cu - Sn - Pb, Cu - Sn - Bi, Cu - Al, Cu - Al - Si, Cu - Si, Cu - Cr, Cu - Pb, Cu - P, et Cu - Te.

10. Procédé selon la revendication 9, dans lequel ledit alliage de cuivre à modifier satisfait à 60 < Cu - 3, 5Si - 1, 8Al - 0,5X + 0,5Y + Mn < 90, où X est Sn, Sb, As ou Mg et Y est Pb, Bi, Se, Te ou Cr.
